# EUROPÄISCHE PATENTSCHRIFT

(11) **EP 0 933 093 B1**
(45) Veröffentlichungstag und Bekanntmachung des Hinweises auf die Patenterteilung: **17.09.2003**
(21) Anmeldenummer: 99101843.3
(22) Anmeldetag: 28.01.1999
(51) Int. Cl.: A61M 15/00

(54) **Inhalator für die Aromatherapie**
Inhalator for aromatherapy
Inhalateur pour la thérapie à l'aide d'essences

(30) Priorität: 29.01.1998 DE 19803376
(43) Veröffentlichungstag der Anmeldung: 04.08.1999
(73) Patentinhaber: Storz, Markus, 78532 Tuttlingen (DE)
(72) Erfinder: Storz, Markus, 78532 Tuttlingen (DE)
(74) Vertreter: Späth, Dieter, Dipl.-Ing.

(56) Entgegenhaltungen:
- EP-A- 0 820 780
- AT-B- 348 661
- CH-A- 201 444
- DE-C- 102 693
- DE-C- 289 881
- DE-U- 29 610 936
- US-A- 1 818 692

## Beschreibung

Die Erfindung betrifft ein Inhalator nach dem Oberbegriff des Anspruchs 1.

Für die Aromatherapie werden z. Zt. die Aroma- und Wirkstoffdämpfe hauptsächlich mittels dreier Verfahren erzeugt, nämlich
1. durch Inhalatoren in denen aroma- und wirkstoffhaltige Flüssigkeiten samt einem entsprechendem Lösungsmittel zu einem Aerosolgemisch verwirbelt und dann eingeatmet werden können.
2. durch Duftlampen in denen wiederum in Lösungsmitteln gelöste aroma- und wirkstoffhaltige Flüssigkeiten durch Verdampfen in die Raumluft gebracht werden.
3. durch Verbrennen bzw. Verschwelen von geeigneten Pflanzen oder deren Produkte (meist Harze). Hierbei wird der bei der Verbrennung entstehende Rauch in die Raumluft abgelassen (z.B. Weihrauch oder Räucherstäbchen) oder mittels Zigarette inhaliert (für Asthmatiker gibt es bei akuten Anfällen spez. Asthmazigaretten mit Heilkräutern).

Alle drei Verfahren sind mit Nachteilen behaftet. So müssen z.B. um aroma- und wirkstoffhaltige Flüssigkeiten zu erzeugen, Pflanzen erst aufwendig extrahiert werden. Die Lösungsmittel, in denen die Flüssigkeiten dann gelöst werden, sind unter Umständen für bestimmte Personen (z.B. Allergiker) nicht geeignet. Beim Räuchern oder Rauchen von Pflanzenmaterial (dazu gehört selbstverständlich auch Tabak) entstehen schädliche Verbrennungsprodukte wie z.B. Teere, Nitrosamine oder sogar rußhaltige Feststoffe, welche kontraindikativ zur therapeutischen Wirkung stehen.

In der Gebrauchsmusteranmeldung (Rollennummer DE 296 10 936 Ul) vom 21.6.96 ist ein Gerät zur Erzeugung von Aroma- und Wirkstoffdämpfen mittels Heißluft beschrieben. Hierbei handelt es sich um einen Heißlufterzeuger welcher ohne geeignete Hilfsmittel (Pfeife) seinen Zweck gar nicht erfüllen kann.

Dieser Inhalator ist in der Handhabung sehr umständlich. So muß z. B. der Heißlufterzeuger für jeden Inhalationszug einund wiederausgeschaltet werden, wobei man genau den richtigen Augenblick zum Inhalieren abpassen muß. Zieht man zu früh, atmet man heiße, aber noch wirkstofflose Luft ein (die Wirkstoffe lösen sich erst nach einigen Sekunden Heißlufteinwirkung), zieht man zu spät, so ist ein Teil der wirkstoffhaltigen Dämpfe nutzlos in die Raumluft geblasen. Außerdem muß man sich beim Einatmen der Förderleistung des Heißluftföns anpassen, was zum Verschlucken und heftigen Hustenanfällen führen kann. Die noch hohe Temperatur des Dampfes stellt ein weiteres Problem dar.

Aus der DE 195 41 528 A1 sowie aus der DE 195 41 690 A1 sind Inhalatoren bekannt, die ein Behältnis zum Sammeln von erzeugten aroma- und/oder wirkstoffhaltigen Dämpfe aufweisen, welches mit dem restlichen Inhalator trennbar verbunden ist. Zu Reinigungszwecken kann das Behältnis abgenommen werden. Während dem Inhalationsbetrieb wird das Behältnis zusammen mit dem restlichen Inhalator als eine Einheit betrieben. Dadurch erweisen sich die beschriebenen Inhalatoren als sehr unhandlich, was den Einsatz gerade bei bettlägerigen oder behinderten Patienten sehr schwierig macht.

Der vorliegenden Erfindung liegt die Aufgabe zugrunde, einen Inhalator nach dem Oberbegriff des Anspruchs 1 so weiterzubilden, daß er die vorgenannten Nachteile möglichst überwindet und insbesondere für die Anwendung bei Bettlägerigen oder Behinderten geeignet ist.

Die Erfindung beruht im Wesentlichen darauf, daß zunächst Heißluft durch das Pflanzenmaterial geblasen wird und dadurch die Aroma- und Wirkstoffe in Dampf übergehen, wobei allerdings die ganze "Portion" in einem Zug verdampft und über ein Ventil in einen Ballon geleitet und gesammelt wird. Aus diesem Ballon kann dann mittels eines auf das Ventil gesetzten Mundstücks der Dampf inhaliert werden.

Die mit der Erfindung erzielten Vorteile bestehen insbesondere darin, daß das zu verdampfende Gut in einem Zug und ohne abzusetzen mit Heißluft durchströmt werden kann. Weiter können dann die im Ballon gesammelten und abgekühlten Dämpfe unabhängig vom Tischgerät ganz bequem und individuell inhaliert werden. Auf diese Art und Weise kann z.B. ein Ballon vom Pflegepersonal abgefüllt und einem bettlägerigen Patienten ohne weiteres verabreicht werden.

Eine Weiterbildung der Erfindung ermöglicht es, einen Tiegel in die Füllkammer zu setzen, aus welchem dann auch ätherische Öle oder andere zweckentsprechende Flüssigkeiten verdampft werden können.

Eine zweite Weiterbildung der Erfindung ermöglicht es, das entstandene Dampf/Luftgemisch aus dem Ballon mittels eines Kompressors wieder abzusaugen, zu verdichten und in einen mit einem Ventil versehenen Druckbehälter abzufüllen, oder die gewonnenen Dämpfe über einen Kompressor unmittelbar in einen Druckbehälter abzufüllen.

Dies hat den Vorteil, daß man sich zu Hause eine entsprechende Portion abfüllen und in einem kleinen, bequem zu transportierenden Behältnis mit sich tragen und bei Bedarf zur Anwendung bringen könnte.

Eine dritte Weiterbildung der Erfindung ermöglicht es, zu verhindern, daß bei zu hoch eingestelltem Temperaturniveau die Kräuter in Brand gesetzt werden können.

Bekannt ist es, daß z.B. bei einem elektronisch geregelten Heißluftfön ein Temperaturfühler den Ist- mit dem Sollwert ständig vergleicht und über die elektronische Steuerung dafür sorgt, daß die Temperatur immer dem eingestellten Niveau entspricht.

Danach wird in das Füllstück oberhalb der Füllkammer ein Rauchdetektor eingebaut, welcher das aufsteigende Dampf/Luftgemisch überwacht und bei entstehendem, unerwünschtem Rauch, welcher durch beginnendes Verschwelen der Kräuter hervorgerufen wird, ein Signal an die Steuerung abgibt, welche daraufhin sofort die Temperatur herunterfährt, um ein Weiterschwelen bzw. Inbrandsetzen der Kräuter zu verhindern.

Ein Ausführungsbeispiel der Erfindung ist in den Zeichnungen dargestellt und wird im folgenden näher beschrieben. Es zeigen:
- Fig. 1:: den Inhalator nach der Erfindung mit aufgesetztem Füllstück und Ventilstück sowie Dampfballon,
- Fig. 2:: den gefüllten Dampfballon mit geschlossenem Ventil und Mundstück unmittelbar vor der Inhalation und
- Fig. 3:: den halbgefüllten Dampfballon mit offenem Ventil und Mundstück während der Inhalation.

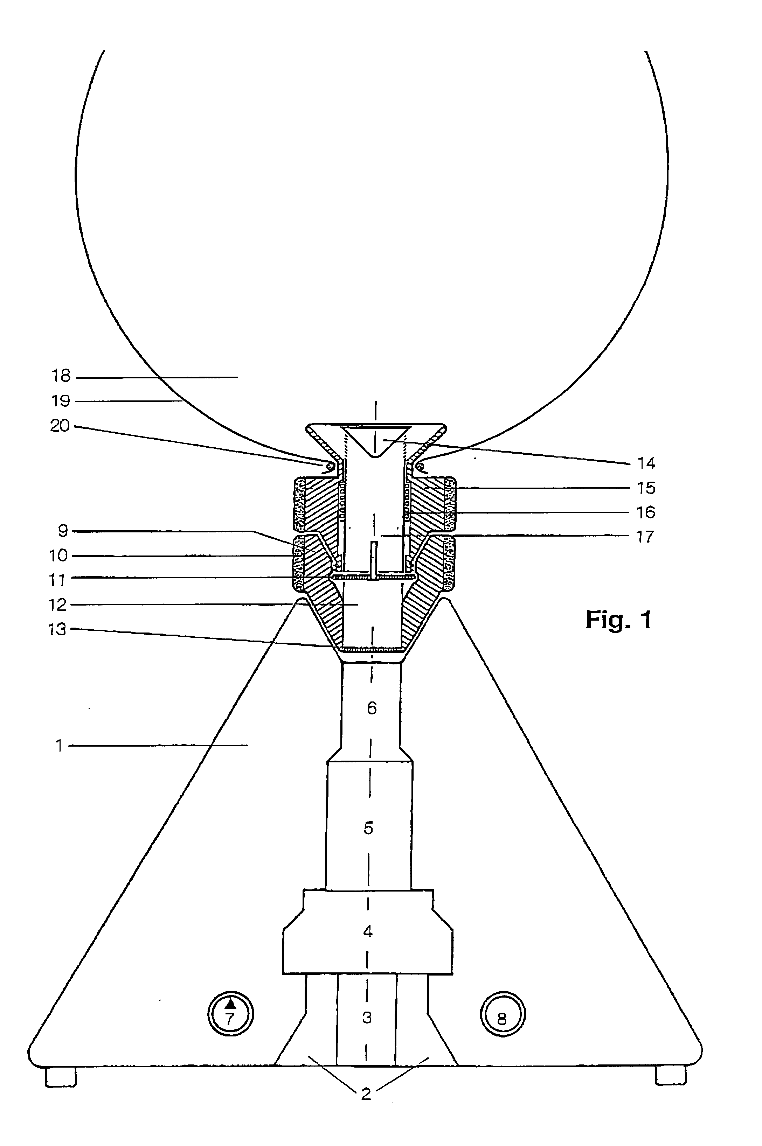

Fig. 1 zeigt das Tischgerät mit aufgesetztem Füllstück 9 und Ventilstück 15 sowie dem Dampfballon 18. Das Gehäuse 1 ist zweckmäßigerweise "Vulkan"-kegelförmig ausgebildet, was der Standfestigkeit des Gerätes zugutekommt. Weiter sorgt die "Vulkan"-Kraterform dafür, daß das aufgesetzte Füllstück 9 ohne Fummelei von selbst an seinen richtigen Platz gleitet. Das gleiche gilt für das in das Füllstück 9 einzusetzende Ventilstück 15. Im Innern des Gehäuses 1 befindet sich ein (schematisch dargestellter) Heißlufterzeuger bestehend aus Motor 3, Propeller 4, Heizkammer 5 und Luftstromrohr 6, welcher die Luft von unten durch einen in der Bodenplatte vorgesehenen Lufteinlaß 2 ansaugt, erhitzt und gemäß dem physikalischem Gesetz, nachdem erhitzte Luft nach oben steigt, aufwärts befördert ("Vulkan"-Schlot).

Im Grunde genommen ist es unwesentlich auf welche Art und Weise die notwendige Heißluft erzeugt wird, denkbar wäre es z.B. eine Glühbirne in die Heizkammer einzubauen, was den Vorteil hätte, daß man aufgrund des nach oben strahlenden Lichtes den Dampf besser erkennen könnte. Es muß allerdings gewährleistet sein, daß der Heißlufterzeuger in der Lage ist, die Luft auf Temperaturen zwischen 50° und 300°C zu bringen. Selbstverständlich dürfen durch die Art und Weise der Heißlufterzeugung keine schädlichen Emmissionen entstehen, die der medizinischen Nutzung des Gerätes entgegenstellen würden. Weiter befinden sich an der Außenseite des Gehäuses 1 ein Temperaturregler 7 sowie ein Ein/Ausschalter 8, welcher als Taster ausgebildet sein muß, so daß nur beim Niederdrücken der Taste der Heißlufterzeuger arbeitet. Das Füllstück 9, vorzugsweise aus Leichtmetall herzustellen, hat eine Füllkammer 12, in die entweder das zu verdampfende Pflanzenmaterial locker eingefüllt oder der Tiegel mit der zu verdampfenden Flüssigkeit eingesetzt wird. Dieser Tiegel (ohne Abbildung) ist stromlinienförmig (wie ein Geschoß mit der Spitze nach unten, oben offen) ausgebildet mit nur ca. halb so großem Durchmesser wie die Füllkammer und wird durch drei oder vier außen an ihm angebrachten Stegen genau in der Mitte der Füllkammer zentriert, so daß er von der Heißluft umströmt und die darin befindliche Flüssigkeit erhitzt und verdampft werden kann.

Unten am Füllstück 9 befindet sich ein mit dem Füllstück fest verbundenes Sieb oder Rost 13, welches verhindert, daß das Füllgut nach unten fällt, jedoch die Heißluft ungehindert durchläßt. Ein weiteres Sieb 11 schließt die Füllkammer nach oben ab, um zu verhindern, daß Pflanzenmaterial nach oben in das Ventilstück 15 geblasen wird. Dieses Sieb ist abnehmbar um ein Befüllen und Entleeren der Füllkammer zu ermöglichen.

Um das Herausnehmen des Siebes zu vereinfachen, ist in der Mitte des Siebes ein nach oben ragender Stift angebracht. Weiter ist das Füllstück wie auch das Ventilstück außen mit einem breiten Gummiring 10 umgeben, welcher eine gute Griffigkeit und Isolierung zum erwärmten Metall hin gewährleistet. Das Füllstück ist zum einfacheren Befüllen und Reinigen genauso wie das Ventilstück abnehmbar und wird nur durch seine konische Formgebung und durch die Schwerkraft am richtigen Platz gehalten. Das Ventilstück 15 besteht aus einem Leicht metallblock in welchem sich ein Ventilzylinder 17 und eine außen um den Ventilzylinder gewundene Ventilfeder 16 befindet.

Fig. 1 zeigt das Ventil in geöffnetem Zustand. Ist die Ventilfeder 16 entspannt, so ist das Ventil geschlossen. Der Ventilzylinder 17 ist durch Stege mit dem Ventildeckel 14 fest verbunden. Die Öffnungen zwischen den Stegen ermöglichen bei geöffnetem Ventil, daß der Dampf in den Ballon hinein- und hinausgelangen kann. Die trichterartige Ausformung des oberen Teils des Ventilstücks 15 sorgt dafür, daß die beim Befüllen zunächst noch schlaffe Ballonhülle 19 nicht unmittelbar mit dem heißen Dampf in Berührung kommt, dessen Temperatur durch die vergrößerte Austrittsoberfläche weiter herabgesetzt wird. Außerdem schützt die Trichterform selbst bei geöffnetem Ventil den Ventildeckel und die zum Zylinder laufenden dünnen Stege vor eventueller Beschädigung, sorgt für eine perfekte Dichtigkeit bei geschlossenem Ventil und hält auch noch den Ballongummi 20 fest an seinem Platz.

Der Dampfballon 18 besteht aus der Ballonhülle 19 und dem Ballonhaltegummi 20. Er sollte in verschiedenen, dem jeweiligen Verwendungszweck angepaßten Größen angeboten werden. Die Ballonhülle besteht aus nichtelastischem, nicht hitzeempfindlichem Kunststoff, der zum Schutz eventuell lichtempfindlicher Inhaltsstoffe dunkel eingefärbt sein kann.

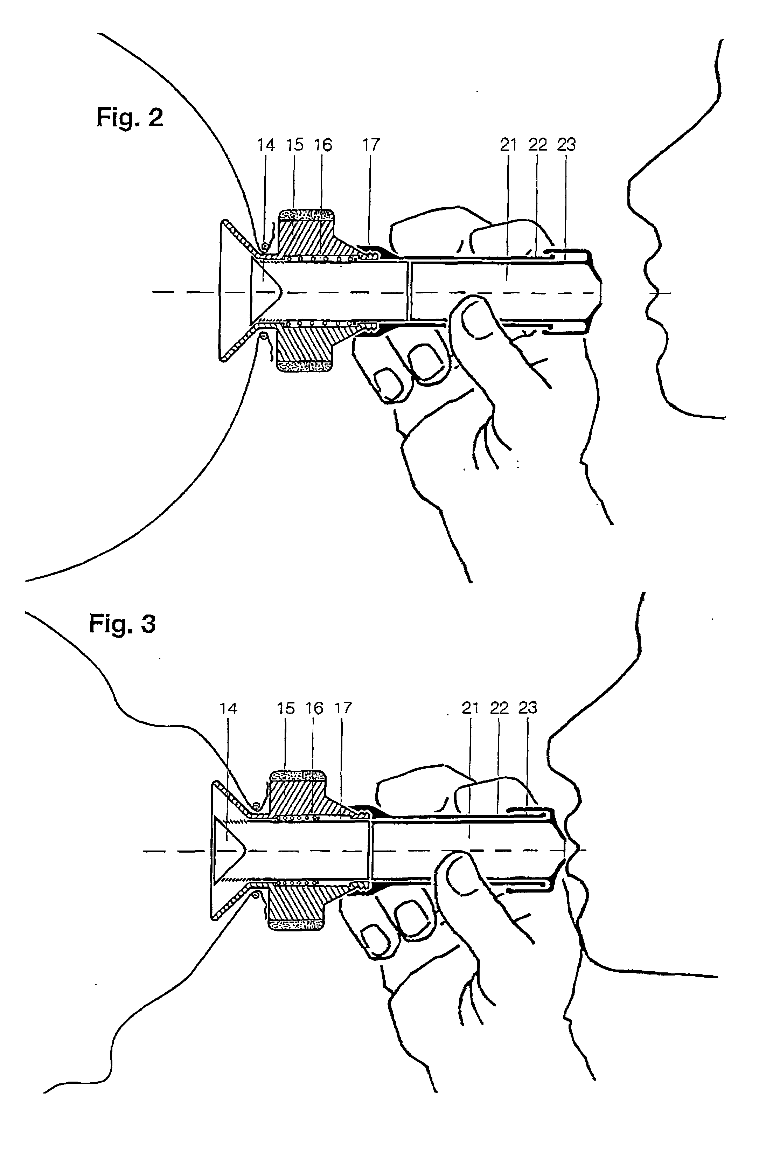

Fig. 2 zeigt den gefüllten Ballon mit Ventilstück (Ventil geschlossen), einem angeschraubten Mundstück 21 sowie einer schematisch dargestellten Person unmittelbar vor der Inhalation. Das Mundstück 21 besteht vorzugsweise aus Kunststoff und ist zweckmäßigerweise zweistückig gestaltet, nämlich einen äußeren Zylinder 22 und einen inneren Zylinder 23. Der äußere Zylinder 22 ist mit dem Ventilstück 15 verbunden. Dies kann durch Verschrauben, wie abgebildet, über einen Bajonettverschluß oder durch simples Aufstecken geschehen. Der innere Zylinder 23 weist ein ausgeformtes Mundstück auf, das im äußeren Zylinder 22 gleitet, den gleichen Durchmesser und das gleiche Verschiebespiel wie der Ventilzylinder 17 aufweist. Der innere Zylinder 23 ist, wie abgebildet, durch eine Überlappung gegen das Herausfallen aus dem äußeren Zylinder 22 gesichert. Die Fig. 3 zeigt, wie das Ventil durch leichten Druck mit den Lippen auf das Mundstück geöffnet wird, worauf der Weg zur Inhalation freigegeben wird.

Im einzelnen läuft die Inhalation wie folgt ab:
1. Zunächst nimmt man das Füllstück und befüllt die Kammer mit dem zerkleinerten Pflanzenmaterial oder dem mit Flüssigkeit gefüllten Tiegel. Dann wird das obere Sieb wieder eingesetzt um zu verhindern, daß das Pflanzenmaterial in das Ventilstück geblasen wird. Jetzt setzt man das Füllstück in den "Vulkan"-Krater.
2. Die notwendige Temperatur wird am Regler eingestellt. Sie ist abhängig vom Feuchtigkeitsgehalt und der Art des Pflanzenmaterials. Je feuchter das Material, umso höher die erforderliche Temperatur. Ist die Temperatur zu niedrig, so findet keine Verdampfung statt; ist sie zu hoch, wird das Pflanzenmaterial in Brand gesetzt.
3. Nun wird der Ein/Ausschalter betätigt. Es vergehen einige Sekunden bis sich die Luft und das Füllgut soweit erwärmt haben, daß eine Verdampfung stattfindet.
4. Sobald man aus dem "Vulkan" Dampf aufsteigen sieht, wird das Ventilstück mit dem daran befestigten, leeren Dampfballon aufgesetzt. Das Ventil wird dabei automatisch geöffnet und nach wenigen Sekunden ist der Ballon gefüllt und wird wieder abgenommen, wobei sich das Ventil automatisch wieder schließt, so daß kein Dampf entweichen kann. Gleichzeitig wird der Finger vom Ein/Ausschalter genommen, das Gerät ist dann abgeschaltet.
5. Jetzt braucht man nur noch das Mundstück auf das Ventilstück aufzuschrauben und kann dann mit der Inhalation beginnen.

Durch leichten Druck mit den Lippen auf das Mundstück wird das Ventil geöffnet und der Balloninhalt kann ganz individuell nach und nach eingeatmet werden.

Es versteht sich von selbst, daß wenn man die Dämpfe in die Raumluft abgeben möchte, kein Ballon mit Ventil aufgesetzt wird. Sollte durch eine Unachtsamkeit der Heißlufterzeuger bei vollständig gefüllten Ballon weiterlaufen, so wird der Ballon zusammen mit dem Ventilstück (unterstützt durch die Kraft der Ventilfeder) durch den Luftdruck angehoben und die überschüssige Luft kann durch den entstehenden Spalt zwischen Ventilund Füllstück entweichen. Auf einen zusätzlichen Sicherheitsmechanismus kann daher verzichtet werden.

Sollte es sich als notwendig erweisen einen Schwebstofffilter einzubauen, so kann dies an folgenden Stellen geschehen:
1. Integriert mit dem oberen Sieb 11.
2. Im Ventilzylinder. Hierbei würde gleich zweimal gefiltert werden; einmal beim Befüllen und das andere mal beim Entleeren des Ballons.
3. Im inneren Zylinder 23 des Mundstücks.

### Bezugszeichenliste

- 1: Gehäuse, Heißlufterzeuger
- 2: Lufteinlaß
- 3: Motor
- 4: Propeller
- 5: Heizkammer
- 6: Luftstromrohr
- 7: Temperaturregler
- 8: Ein/Ausschalter
- 9: Füllstück
- 10: Gummiring
- 11: Sieb
- 12: Füllkammer
- 13: Rost
- 14: Ventildeckel
- 15: Ventilstück
- 16: Ventilfeder
- 17: Ventilzylinder
- 18: Dampfballon, Behältnis
- 19: Ballonhülle
- 20: Ballonhaltegummi
- 21: Mundstück
- 22: Äußerer Zylinder
- 23: Innerer Zylinder

## Patentansprüche

1. Inhalator zur Erzeugung von aroma- und/oder wirkstoff haltigen Dämpfen, mit einem Dampferzeuger (11 bis 13) und einem mit diesem verbundenen, abnehmbaren Behältnis (18) zum Sammeln der Dämpfe,
**dadurch gekennzeichnet,**
**daß** die Dämpfe unter Einsatz einer Heißluftquelle (1) erzeugbar sind, daß das Behältnis (18) mit wenigstens einer Öffnung zur Zuführung und Entnahme der Dämpfe versehen ist und dass diese wenigstens eine Öffnung mittels eines Ventilmechanismus (15 bis 17) verschließbar ist.

2. Inhalator nach Anspruch 1,
**dadurch gekennzeichnet,**
**daß** das Gehäuse (1) des Dampferzeugers (11 bis 13) einem Vulkan ähnelt.

3. Inhalator nach Anspruch 1 oder 2,
**dadurch gekennzeichnet,**
**daß** ein Tiegel vorgesehen ist, durch welchen sich Flüssigkeit verdampfen läßt.

4. Inhalator nach einem der Ansprüche 1 bis 3,
**dadurch gekennzeichnet,**
**daß** ein Füllstück (9) vorgesehen ist, welches mit dem am Behältnis (18) befindlichen Ventilmechanismus (15) zusammenwirkt und auf dieses aufsteckbar und lösbar ist.

5. Inhalator nach einem der Ansprüche 1 bis 4,
**dadurch gekennzeichnet,**
**daß** Füllstück (9) und Ventilstück (15) beim Aufsetzen in den jeweiligen Krater allein durch ihre konische Formgebung und die Schwerkraft vollends von selbst an ihren vorgesehenen Platz gleiten.

6. Inhalator nach einem der Ansprüche 1 bis 5,
**dadurch gekennzeichnet,**
**daß** die Austrittsöffnung des Ventilstücks (15) und eines Ventildeckels (14) trichterförmig ausgebildet sind.

7. Inhalator nach einem der Ansprüche 1 bis 6,
**dadurch gekennzeichnet,**
**daß** ein Mundstück (21) vorgesehen ist, das aus einem äußeren Zylinder (22) besteht, der die Verbindung zum Ventilstück (15) herstellt, sowie einem zweiten inneren Zylinder (23), durch welchen der Druck der Lippen einer Bedienperson auf den Ventilmechanismus (15) übertragbar ist.

8. Inhalator nach Anspruch 1,
**dadurch gekennzeichnet,**
**daß** das Behältnis (18) als Kunststoffbeutel ausgebildet ist.

9. Inhalator nach Anspruch 1,
**dadurch gekennzeichnet,**
**daß** ein Kompressor vorgesehen ist, der zur Befüllung des Behältnisses (18) verwendet wird.

10. Inhalator nach Anspruch 1,
**dadurch gekennzeichnet,**
**daß** ein Rauchdetektor vorgesehen ist, welcher zusammen mit einer Steuerung die Temperatur der Heißluftquelle (1) regelt.

## Claims

1. Inhaler for production of aroma- and/or active substance-containing vapors, with a vaporizer (11 to 13) and a therewith connected, removable container (18) for collection of the vapors, **characterized in that** the vapors are produceable by using a hot air source (1), that the container (18) is provided with at least one opening for introduction and removal of the vapors an that this at least one opening is closable by means of a valve mechanism (15 to 17).

2. Inhaler according to claim 1, **characterized in that** the housing (1) of the vaporizor (11 to 13) has the general shape of a volcano.

3. Inhaler according to claim 1 or 2, **characterized in that** a crucible is provided via which a fluid can be vaporized.

4. Inhaler according to one of claims 1 to 3, **characterized in that** a receptacle (9) is provided, which cooperates with the valve mechanism (15) provided on the container (18) and is upon this seatable and removable.

5. Inhaler according to one of claims 1 to 4, **characterized in that** the receptacle (9) and valve (15), during seating in the respective crater, slide by themselves to their predetermined place as a result of their conical shape and by gravity.

6. Inhaler according to one of claims 1 to 5, **characterized in that** the exhaust opening of the valve (15) and the valve cover (14) are formed funnel-shaped.

7. Inhaler according to one of claims 1 to 6, **characterized in that** a mouthpiece (21) is provided, which is comprised of an outer cylinder (22), which establishes the connection with the valve (15), as well as a second inner cylinder (23), through which the pressure of the lips of an operating person is transmittable to the valve mechanism (15).

8. Inhaler according to claim 1, **characterized in that** the container (18) is a plastic bag.

9. Inhaler according to claim 1, **characterized in that** a compressor is provided, which is utilized for filling the container (18).

10. Inhaler according to claim 1, **characterized in that** a smoke detector is provided, which together with a controller regulates the temperature of the hot air source (1).

## Revendications

1. Inhalateur pour la confection de vapeurs contenant des substances aromatiques et/ou des principes actifs avec générateur de vapeur (11 à 13) et un récipient amovible (18) raccordé à celui-ci pour recueillir la vapeur.
**caractérisé en ceci**
**que** les vapeurs sont créées par une source d'air chaud (1), que le récipient (18) est pourvu d'au moins une ouverture pour l'injection et le prélèvement des vapeurs et que cette ouverture être fermée par un mécanisme de soupape (15 à 17).

2. Inhalateur selon la revendication 1,
**caractérisé en ceci**
**que** le carter (1) du générateur de vapeur (11 à 13) a la forme d'un volcan.

3. Inhalateur selon la revendication 1 ou 2,
**caractérisé en ceci**
**qu**'un creuset est prévu dans lequel le liquide vaporise.

4. Inhalateur selon l'une des revendications 1 à 3,
**caractérisé en ceci**
**qu'**une pièce de remplissage (9) est prévue qui fonctionne de conserve avec le mécanisme de soupape (15) fixé sur le récipient (18) et qui est encastrée sur celui-ci et en est détachable.

5. Inhalateur selon l'une des revendications 1 à 4,
**caractérisé en ceci**
**que** la pièce de remplissage (9) et la pièce de soupape (15) viennent prendre place automatiquement dans le logement qui leur est réservé uniquement par leur forme conique et sous l'effet de la pesanteur.

6. Inhalateur selon l'une des revendications 1 à 5,
**caractérisé en ceci**
**que** l'orifice de sortie de la pièce de soupape (15) et d'un tiroir de soupape (14) a la forme d'un entonnoir.

7. Inhalateur selon l'une des revendications 1 à 6,
**caractérisé en ceci**
**qu**'une pièce buccale (21) est prévue, constituée d'un cylindre extérieur (22) qui établit la liaison avec la pièce de soupape (15) et un second cylindre, intérieur (23), grâce auquel le mouvement provoqué par la pression des lèvres de l'opérateur est transmis au mécanisme de soupape (15).

8. Inhalateur selon la revendication 1,
**caractérisé en ceci**
**que** le récipient (18) est un sac plastique.

9. Inhalateur selon la revendication 1,
**caractérisé en ceci**
**qu'**un compresseur est prévu pour le remplissage du récipient (18).

10. Inhalateur selon la revendication 1,
**caractérisé en ceci**
**qu**'un détecteur de fumée est prévu, réglant la température de la source d'air chaud (1) en association avec la commande.
